# EUROPÄISCHE PATENTSCHRIFT

(11) **EP 0 748 208 B1**
(45) Veröffentlichungstag und Bekanntmachung des Hinweises auf die Patenterteilung: **21.04.1999**
(21) Anmeldenummer: 95909764.3
(22) Anmeldetag: 22.02.1995
(51) Int. Cl.: A61K 7/48, A61K 7/06, A61K 47/14

(54) **KOSMETISCHE UND/ODER PHARMAZEUTISCHE ZUBEREITUNGEN**
COSMETIC AND/OR PHARMACEUTICAL PREPARATIONS
COMPOSITIONS COSMETIQUES ET/OU PHARMACEUTIQUES

(30) Priorität: 03.03.1994 DE 4407015
(43) Veröffentlichungstag der Anmeldung: 18.12.1996
(73) Patentinhaber: Henkel Kommanditgesellschaft auf Aktien, 40191 Düsseldorf (DE)
(72) Erfinder: THOMAS, Heike, 40764 Langenfeld (DE); ANSMANN, Achim, 40699 Erkrath (DE); KAWA, Rolf, 40789 Monheim (DE); WADLE, Armin, 40724 Hilden (DE); BUNTE, Reinhard, 41542 Dormagen (DE); HEES, Udo, 47269 Duisburg (DE); WESTFECHTEL, Alfred, 40723 Hilden (DE)
(86) Internationale Anmeldenummer: EP9500633
(87) Internationale Veröffentlichungsnummer: WO9523586

(56) Entgegenhaltungen:
- EP-A- 0 203 831
- EP-A- 0 379 658
- EP-A- 0 451 461
- EP-A- 0 579 159
- WO-A-94/09753
- WO-A-94/18943
- DE-A- 4 100 490
- DE-A- 4 309 390
- US-A- 3 936 391

## Beschreibung

### Gebiet der Erfindung

Die Erfindung betrifft kosmetische und/oder pharmazeutische Zubereitungen mit erhöhter Viskosität und verbesserter Lagerbeständigkeit, die sich durch einen Gehalt an ausgewählten Palmitinsäureestern von Oligoglycerinen als Emulgatoren auszeichnen.

### Stand der Technik

Zur dauerhaften homogenen Vermischung von ansonsten nicht miteinander mischbaren Stoffen werden Emulgatoren benötigt. Im Bereich kosmetischer und pharmazeutischer Zubereitungen, beispielsweise zur Herstellung von Cremes und Lotionen, kommen vielfach Ester von Fettsäuren mit mehrwertigen Alkoholen wie beispielsweise Pentaerythrit, Dipentaerythrit oder Eigenkondensationsprodukten des Glycerins, sogenannten technischen Oligoglyceringemischen zum Einsatz. Eine Übersicht zu diesem Thema von G.Schuster und H.Pospischil findet sich in **Ärztl. Kosmetol., 11, 30-37 (1981)**. Der Einsatz von Polyglycerinestern als O/W-Emulgatoren für kosmetische Zubereitungen wird beispielsweise in **J.Soc.Cosmet.Chem. 28, 733-740 (1977)** sowie in **Fette, Seifen, Anstrichmittel 88, 101-106 (1986)** beschrieben. Die Verwendung ausgewählter Polyglycerinfettsäureester als kosmetische Emulgatoren wird ferner auch in den Deutschen Offenlegungsschriften **DE-A1 4005819** und **DE-A1 4023593** (BASF) beansprucht. Bei Einsatz der in diesen Schriften genannten Ester auf Basis ungesättigter bzw. gesättigter Fettsäuren beobachtet man jedoch, daß die resultierenden Emulsionen nicht immer ausreichend lagerstabil und/ oder dünnflüssig sind, also eine nicht ausreichend hohe Viskosität besitzen, so daß eine problemlose Dosierung erschwert ist.

Die Aufgabe der Erfindung hat somit darin bestanden, neue Emulgatoren auf Basis von technischen Oligoglyceringemischen und Fettsäuren zur Verfügung zu stellen, die frei von den geschilderten Nachteilen sind.

### Beschreibung der Erfindung

Gegenstand der Erfindung sind kosmetische und pharmazeutische Zubereitungen, die als Emulgatoren Ester der Palmitinsäure mit technischen Triglycerinen mit der Maßgabe enthalten, daß der Gehalt an Monoestern 30 bis 50 Gew.-% beträgt.

Überraschenderweise wurde gefunden, daß der Eigenkondensationsgrad der Oligoglycerine in Verbindung mit der Natur der Fettsäure und dem Anteil an Monoestern einen entscheidenden Einfluß auf die Eigenschaften der resultierenden Emulgatoren besitzt. Die Erfindung schließt dabei die Erkenntnis ein, daß die Einstellung eines Monoesteranteils von 30 bis 50 % in den erfindungsgemäßen Emulgatoren gegenüber ansonsten bekannten Produkten des Standes der Technik zu einer signifikanten Verbesserung der Lagerbeständigkeit und Viskosität führt. Demzufolge führt der Einsatz der erfindungsgemäßen Emulgatoren zu homogenen kosmetischen bzw. pharmazeutischen Zubereitungen vom Typ der O/W-Emulsionen, die über lange Zeit lagerstabil sind, sich nicht entmischen und eine vorteilhaft hohe und dabei konstante Viskosität aufweisen.

### Triglycerinpalmitinsäureester

Ester von technischem Triglycerin mit Fettsäuren stellen bekannte Stoffe dar, die nach den einschlägigen Verfahren der präparativen organischen Chemie erhalten werden können. So kann man gemäß der bereits genannten Druckschriften **DE-A1 4005819** und **DE-A1 4023593** technisches Triglycerin mit Fettsäuren in Gegenwart von hypophosphoriger Säure bei 180°C verestern und das Reaktionswasser dabei kontinuierlich aus dem Gleichgewicht abdestillieren. Die Triglycerinpalmitinsäureester der Erfindung stellen statistisch gesehen Monoester, genauer, Ester von im Durchschnitt 1 Mol technischem Triglycerin mit 1 Mol Fettsäure dar. Hieraus kann folgende typische Verteilung resultieren:

| | |
|---|---|
| Monoester | 30 bis 50 Gew.-% |
| Diester | 30 bis 45 Gew.-% |
| Triester | 15 bis 30 Gew.-% |

Die Emulgatoren können sich auch von Triglycerin der folgenden Zusammensetzung ableiten:

| | |
|---|---|
| Monoglycerin | 0 bis 20 Gew.-% |
| Diglycerin | 10 bis 35 Gew.-% |
| Triglycerin | 10 bis 52 Gew.-% |
| Tetraglycerin | 5 bis 25 Gew.-% |
| Pentaglycerin | 0 bis 10 Gew.-% |
| Hexaglycerin | 0 bis 10 Gew.-% |
| Oligoglycerine | 0 bis 10 Gew.-% |

Triglycerinfettsäureester, die im Sinne der Erfindung besonders vorteilhafte Eigenschaften aufweisen, zeichnen sich durch mindestens eine der beiden folgenden Eigenschaften aus:
- Monoestergehalt im Bereich von 30 bis 50, vorzugsweise 35 bis 50 Gew.-%;
- Triglyceringehalt im Bereich von 10 bis 55, vorzugsweise 35 bis 55 Gew.-% - bezogen auf den Oligoglycerinanteil.

Im Sinne der Erfindung sind als Emulgatoren solche Palmitinsäureester besonders bevorzugt, die einen Monoestergehalt im Bereich von 35 bis 50 Gew.-% und einen Triglyceringehalt im Bereich von 35 bis 55 Gew.-% - bezogen auf den Oligoglycerinanteil - aufweisen. Die Emulgatoren können in den kosmetischen und/oder pharmazeutischen Zubereitungen in Mengen von 0,1 bis 20, vorzugsweise 1 bis 15 Gew.-% - bezogen auf die Zubereitungen - enthalten sein.

### Kosmetische und/oder pharmazeutische Zubereitungen

Die Zubereitungen können in untergeordneten Mengen weitere, mit den anderen Inhaltsstoffen kompatible **Tenside** enthalten. Typische Beispiele sind Fettalkoholsulfate, Fettalkoholpolyglycolethersulfate, Monoglyceridsulfate, Ethercarbonsäuren, Mono- und/oder Dialkylsulfosuccinate, Fett-säureisethionate, Fettsäuridesarcosinate, Fettsäuretauride, Alkyl- und/oder Alkenyloligoglucoside, Alkylamidobetaine und/oder Proteinhydrolysate bzw. Proteinfettsäurekondensate auf tierischer, insbesondere auf pflanzlicher Basis.

Als Hilfs- und Zusatzstoffe kommen ferner Ölkörper, Co-Emulgatoren, Fette und Wachse, Verdickungsmittel, biogene Wirkstoffe, Filmbildner, Duftstoffe, Farbstoffe, Perlglanzmittel, Konservierungsmittel und pH-Regulatoren in Frage.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₁₆-C₁₈-Fettalkoholen, Ester von linearen C₁₀-C₁₈-Fettsäuren mit verzweigten Alkoholen, insbesondere 2-Ethylhexanol, Ester von linearen und/oder verzweigten Fettsäuren mit zweiwertigen Alkoholen und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate und/oder Dialkylether in Betracht.

Als **Co-Emulgatoren** kommen sowohl bekannte W/O- als auch O/W- Emulgatoren in Frage. Typische Beispiele für **Fette** sind Glyceride, als **Wachse** kommen u.a. Bienenwachs, Paraffinwachs oder Mikrowachse in Frage. Geeignete **Verdickungsmittel** sind beispielsweise vernetzte Polyacrylsäuren und deren Derivate, Polysaccharide, insbesondere Xanthan-Gum, Guar-Guar, Agar- Agar, Alginate und Tylosen, Carboxymethylcellulose und Hydroxyethylcellulose, ferner Fettalkohole, Monoglyceride und Fettsäuren, Polyacrylate, Polyvinylalkohol und Polyvinylpyrrolidon. Unter **biogenen Wirkstoffen** sind beispielsweise Pflanzenextrakte, Eiweißhydrolysate und Vitaminkomplexe zu verstehen. Gebräuchliche Filmbildner sind beispielsweise Polyvinylpyrrolidon, Vinylpyrrolidon-Vinylacetat-Copolymerisate, Polymere der Acrylsäurereihe, quaternäre Cellulose- Derivate und ähnliche Verbindungen. Als **Konservierungsmittel** eignen sich beispielsweise Formaldehydlösung, p-Hydroxybenzoat oder Sorbinsäure. Als **Perlglanzmittel** kommen beispielsweise Glycoldistearinsäureester wie Ethylenglycoldistearat, aber auch Fettsäuren und Fettsäuremonoglycolester in Betracht. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, veröffentlicht im Verlag Chemie, Weinheim, 1984,** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Zubereitungen - betragen. Die Herstellung der Mittel kann in an sich bekannter Weise, d.h. beispielsweise durch Heiß-, Kalt-, Heiß- Heiß/Kalt- bzw. PIT-Emulgierung erfolgen. Hierbei handelt es sich um ein rein mechanisches Verfahren, eine chemische Reaktion findet nicht statt. Die kosmetische und/oder pharmazeutischen Zubereitungen können einen Wasseranteil von 25 bis 95, vorzugsweise 50 bis 75 Gew.-% aufweisen.

### Beispiele

**Triglycerinpalmitate.** Die Triglycerinpalmitate der Erfindung können in an sich bekannter Weise durch Veresterung von technischen Triglyceringemischen mit Palmitinsäure in Gegenwart alkalischer Katalysatoren erhalten werden. Die Zusammensetzung der erfindungsgemäßen Emulgatoren ist in Tabelle 1 wiedergegeben.

**Tabelle 1**

| **Zusammensetzung der erfindungsgemäßen Emulgatoren (Mengenangaben als Gew.-%)** | | | | | | | | | | |
|---|---|---|---|---|---|---|---|---|---|---|
| **Emul.** | **Oligoglycerinteil** | | | | | | | **Esterteil** | | |
| | **MG** | **DG** | **TG** | **tG** | **PG** | **HG** | **OG** | **ME** | **DE** | **TE** |
| A1 | 18 | 27 | 21 | 14 | 9 | 4 | 7 | 39 | 33 | 28 |
| A2 | 0 | 24 | 52 | 16 | 5 | 2 | 1 | 39 | 35 | 26 |
| A3 | 18 | 27 | 21 | 14 | 9 | 4 | 7 | 44 | 34 | 22 |

| | | | | | | | | | | |
|---|---|---|---|---|---|---|---|---|---|---|
| Legende: Emul. = Emulgator | | | | | | | | | | |
| MG = Monoglycerin | | | | | | | | | | |
| DG = Diglycerin | | | | | | | | | | |
| TG = Triglycerin | | | | | | | | | | |
| tG = Tetraglycerin | | | | | | | | | | |
| PG = Pentaglycerin | | | | | | | | | | |
| HG = Hexaglycerin | | | | | | | | | | |
| OG = Oligoglycerin | | | | | | | | | | |
| ME = Monoester | | | | | | | | | | |
| DE = Diester | | | | | | | | | | |
| TE = Triester | | | | | | | | | | |

**Anwendungstechnische Untersuchungen.** Die anwendungstechnische Beurteilung der Emulsion wurde an Hand der Basisrezepturen gemäß Tabelle 2 durchgeführt .

**Tabelle 2**

| **Basisrezepturen (Mengenangaben als Gew.-%)** | | | | | |
|---|---|---|---|---|---|
| **Zusammensetzung (INCI Bezeichnung)** | **R1** | **R2** | **R3** | **R4** | **R5** |
| Emulgator | 6 | 3 | 16 | 16 | 16 |
| Dodecyl Oleate | 10 | 10 | - | - | - |
| Capric Caprylic Triglyceride | - | - | 16 | - | - |
| Cetearyl Isononanoate | - | - | - | 16 | - |
| Paraffin Oil | - | - | - | - | 16 |
| Glyceryl Stearate | - | 5 | - | - | - |
| Wasser | ad 100 | | | | |

Die Viskosität der Emulsionen wurde nach einer Woche bei 23°C in einem Brookfield RVF-Viskosimeter, Spindel TE, 4 UpM gemessen und die Stabilität nach einer Lagerung von 4 Wochen bei 20°C und 40°C visuell beurteilt. Die Ergebnisse sind in Tabelle 3 zusammengefaßt. Die Beispiele 1 bis 7 sind erfindungsgemäß, die Beispiele V1 bis V8 dienen zum Vergleich.

**Tabelle 3**

| **Anwendungstechnische Beurteilung der Emulsionen** | | | | |
|---|---|---|---|---|
| **Bsp.** | **R** | **Emulgator** | **Viskosität [mPas]** | **Stabilität** |
| 1 | 1 | A1 | 31.250 | stabil |
| 2 | 2 | A1 | 200.000 | stabil |
| 3 | 2 | A2 | 137.500 | stabil |
| 4 | 2 | A3 | 275.000 | stabil |
| 5 | 3 | A1 | 225.000 | stabil |
| 6 | 4 | A1 | 212.000 | stabil |
| 7 | 5 | A1 | 350.000 | stabil |
| V1 | 1 | Ceteareth-12 | - | instabil |
| V2 | 1 | Triglycerinmonostearat mit 90 % Monoester (analog DE 4023593) | - | instabil |
| V3 | 2 | Triglycerindistearat | 100.000 | stabil |
| V4 | 2 | Triglycerinmonostearat mit 90 % Monoester (analog DE 4023593) | 18.750 | instabil |
| V5 | 2 | Ceteareth-12/Ceteareth-20 (1:1) | 2.000 | stabil |
| V6 | 3 | Triglycerinmonomyristat (42 % Monoester) | 25.000 | instabil |
| V7 | 4 | Triglycerinmonomyristat (42 % Monoester) | 12.500 | instabil |
| V8 | 5 | Triglycerinmonomyristat (42 % Monoester) | 25.000 | instabil |

Die Beispiele zeigen, daß der Übergang zu höheren Monoestergehalten oder kürzerkettigen Fettsäureresten zu einer signifikanten Verschlechterung der anwendungstechnischen Eigenschaften der Emulgatoren führt.

## Patentansprüche

1. Kosmetische und pharmazeutische Zubereitungen, **dadurch gekennzeichnet**, daß sie als Emulgatoren Ester der Palmitinsäure mit technischen Triglycerinen mit der Maßgabe enthalten, daß der Gehalt an Monoestern 30 bis 50 Gew.-% beträgt.

## Claims

1. Cosmetic and pharmaceutical formulations, characterized in that they contain esters of palmitic acid with technical triglycerols as emulsifiers, with the proviso that the monoester content is 30 to 50% by weight.

## Revendications

1. Préparations cosmétiques et pharmaceutiques
caractérisées en ce qu'
elles contiennent comme émulsifiants des esters de l'acide palmitique avec des triglycérides industriels, sous réserve que la teneur en monoesters s'élève à 30 à 50 % en poids.
